# EUROPEAN PATENT APPLICATION

(11) **EP 4 763 102 A1**
(43) Date of publication of application: **24.06.2026**
(21) Application number: 25217098.0
(22) Date of filing: 19.11.2025
(51) Int. Cl.: A61B 17/12

(54) **OCCLUDER DEVICES**

(30) Priority: 19.12.2024 US 202463736059 P; 04.04.2025 US 202563783385 P
(71) Applicant: St. Jude Medical, Cardiology Division, Inc., St. Paul, MN 55117 (US)
(72) Inventor: MORIN, Kristen T., St. Paul, 55105 (US); MEYER, Michael P., Minnetrista, 55359 (US); ERZBERGER, Gary, Corcoran, 55340 (US); ASLESON, Andrea, Maple Grove, 55311 (US); EIDENSCHINK, Tracee, Wayzata, 55391 (US)
(74) Representative: Gill Jennings & Every LLP

(57) **Abstract**

A medical device for occluding a left atrial appendage, LAA, the medical device comprising a single occlusive member configured to occlude an ostium of the LAA in a deployed condition of the medical device, wherein the occlusive member is a disc-shaped portion, and wherein the medical device is configured to be attached only to the ostium of the LAA.

## Description

### CROSS-REFERENCE TO RELATED APPLICATIONS

This application claims priority to U.S. Provisional Patent Application No. 63/736,059, filed December 19, 2024, and U.S. Provisional Patent Application No. 63/783,385, filed April 4, 2025.

### FIELD OF THE DISCLOSURE

The present disclosure relates generally to medical devices that are used in the human body. In particular, the present disclosure is directed to embodiments of occluder devices and associated methods of manufacturing and operation.

### BACKGROUND OF THE DISCLOSURE

An occluder is a medical device used to treat (e.g., occlude) tissue at a target site within the human body, such as an abnormality, a vessel, an organ, an opening, a chamber, a channel, a hole, a cavity, a lumen, or the like. For example, an occluder may be used to occlude or close an atrial septal defect (ASD), a ventricular septal defect (VSD), or a patent foramen ovale (PFO), which are generally characterized as holes in the subject tissue.

In other examples, an occluder may be used for Left Atrial Appendage ("LAA") closures. An LAA is a normal anatomical structure in which there is a sac in the muscle wall of the left atrium. When a patient experiences atrial fibrillation ("AFib"), a blood clot may be formed within the LAA which may become dislodged and enter into the blood stream. By occluding the LAA, the release of blood clots from the LAA may be significantly reduced, if not eliminated. Various techniques have been developed to occlude the LAA.

### SUMMARY OF THE DISCLOSURE

According to a first aspect of the disclosure there is provided a medical device for occluding a left atrial appendage, LAA, the medical device comprising a single occlusive member configured to occlude an ostium of the LAA in a deployed condition of the medical device, wherein the occlusive member is a disc-shaped portion, and wherein the medical device is configured to be attached only to the ostium of the LAA.

The occlusive member (which may be interchangeably referred to herein as an occluder member, an occluding member, or simply an occluder) may be a combined occlusive and fixing portion. The occlusive member may comprise a frame and an occlusive braided fabric. The occlusive member may consist of an occlusive braided fabric. The occlusive braided fabric may comprise a shape-memory alloy. The occlusive braided fabric may consist of only the single disc-shaped portion. The device may be configured for long-term treatment. The medical device may comprise fixing elements for attaching the occlusive member to the target site. The plurality of fixing elements may be provided at a periphery of the occlusive member. Each fixing element may extend from the occlusive member in a fixing direction and at a respective fixing location on the occlusive member. The fixing direction may have an outward component, extending in an outward direction from the occlusive member at the location, and a tangential component, extending in a tangential direction to the occlusive member at the location. Each fixing element may be arranged such that rotation of the medical device in a first direction causes the plurality of fixing elements to engage with the tissue at the target site. The rotation may be about an axial direction of the medical device. The plurality of fixing elements may be configured to oppose disengagement from the tissue. The fixing elements may each comprise a tine, barb or hook. The fixing elements may be configured to directly couple the occluder member to tissue at the target site at the locations of the fixing elements. The fixing elements may be coupled to the frame of the occluder member. The plurality of fixing elements may comprise a first set of fixing elements and a second set of fixing elements. The first set of fixing elements may oppose the second set of fixing elements. A tangential component of the fixing direction of the first set of fixing elements may oppose a tangential component of the fixing direction of the second set of fixing elements. The first set of fixing elements may extend in a clockwise direction. The second set of fixing elements may extend in an anticlockwise direction. The second set of fixing elements may be displaced from the first set of fixing elements. The second set of fixing elements may be displaced from the first set of fixing elements in the longitudinal direction.

The medical device may also comprise an installation structure. The installation structure may comprise a self-expanding body extending between a proximal end and a distal end. In a deployment configuration of the medical device, the occlusive member may be releasably coupled to the distal end of the installation structure. The self-expanding body may comprise a balloon. The self-expanding body may comprise a plurality of struts. The installation structure may comprise a deployment mechanism for an adhesive. The deployment mechanism may be arranged to deposit deployed adhesive to couple the medical device to tissue at the target site.

The medical device may also comprise a tether coupled to a distal end of the medical device. The tether may be configured to anchor the medical device in a distal wall of the LAA.

The medical device may also comprise one or more electrodes for bonding the medical device to tissue at the target site. The one or more electrodes may be coupled to the occluding member. The one or more electrodes may be electrically insulated from the occluding member. The medical device may also comprise one or more electrical leads coupled to the one or more electrodes for delivering energy to the one or more electrodes. The one or more electrical leads may each comprise a sacrificial portion to provide a point for detaching the one or more electrical leads from the one or more electrodes. The one or more electrodes may be arranged to interface with tissue at the target site. The one or more electrodes may be configured to receive energy to cause the one or more electrodes to be heated such that the medical device is bonded to the tissue at, or by, the one or more electrodes. The one or more electrodes may each comprise an insulating coating. The one or more electrodes may each comprise a first side in contact with the occluding member and a second side configured to interface with the tissue. At least a portion of the second side of each of the one or more electrodes may be free of insulating material. The occluding member may comprise an occlusive braided fabric. The one or more electrodes may each comprise a wire segment wrapped around one or more strands of the braided fabric.

According to a further aspect of the disclosure there is provided a method for deploying a medical device at an ostium of an LAA using a delivery device, wherein the delivery device includes a delivery cable having a distal end coupled to the proximal end of the medical device, the method comprising: positioning an occluding disc of the medical device against the ostium; rotating the medical device by rotating the delivery cable to cause fixing elements of the medical device to engage with tissue at the target site to secure the medical device to the ostium; decoupling the disc of the medical device from the delivery device; and removing the delivery device and leaving the medical device in situ at the ostium of the LAA.

The delivery device may also include an expandable installation structure. The distal end of the delivery cable may be coupled to the proximal end of the medical device via the expandable installation structure. The method may also comprise deploying the installation structure from a delivery catheter. Deploying the installation structure may include expanding the installation structure from a constricted delivery configuration to an expanded deployment configuration and simultaneously expanding the disc coupled to the installation structure. The expandable installation structure may comprise a balloon. The expandable installation structure may comprise a self-expanding installation frame. Deploying the installation structure may comprise retracting the delivery catheter, and/or distally advancing the delivery cable through the delivery catheter. Securing the medical device may also comprise affixing, suturing, adhering, bonding or stapling the disc to the tissue.

According to a further aspect of the disclosure there is provided a method for deploying a medical device comprising an occluding member for treating a target site, using a delivery device, wherein the delivery device includes a delivery cable having a distal end coupled to a proximal end of the medical device, wherein the medical device comprises one or more electrodes for bonding the medical device to tissue at the target site, wherein the one or more electrodes are coupled to the occluding member, and wherein the one or more electrodes are electrically insulated from the occluding member, the method comprising: positioning the occluding member of the medical device at the target site; delivering, via the delivery device, energy to the one or more electrodes to cause heating of the one or more electrodes for causing the tissue to bond to the one or more electrodes to secure the medical device to the target site; decoupling the medical device from the delivery device; and removing the delivery device and leaving the medical device in situ at the ostium of the LAA.

A method for deploying a medical device comprising an occluder disc at an ostium of an LAA using a delivery device, wherein the delivery device includes a delivery cable having a distal end coupled to a proximal end of the medical device, wherein the delivery device further comprises an expandable installation structure comprising a balloon, and wherein, in a constructed delivery configuration, the distal end of the delivery cable is coupled to the proximal end of the medical device via the expandable installation structure, the method comprising: deploying the occluder disc from the delivery catheter adjacent to the ostium; deploying the installation structure from the delivery catheter, expanding the balloon from the constricted delivery configuration to an expanded deployment configuration and simultaneously expanding the occluder disc coupled to the installation structure; securing the expanded occluder disc to the ostium; decoupling the occluder disc of the medical device from the delivery device; and removing the delivery device and leaving the medical device in situ at the ostium of the LAA.

According to a further aspect of the disclosure there is provided a medical device for treating a target site, the device comprising: a body; and a plurality of fixing elements for coupling the body of the medical device to tissue at the target site, wherein each fixing element extends from the body in a fixing direction and at a respective fixing location on the body, wherein the fixing direction has an outward component, extending in an outward direction from the body at the location, and a tangential component, extending in a tangential direction to the body at the location, such that rotation of the body causes the plurality of fixing elements to engage with the tissue at the target site.

The plurality of fixing element may be provided at a periphery of the occlusive member (or the body thereof). Each fixing element may be arranged such that rotation of the medical device in a first direction causes the plurality of fixing elements to engage with the tissue at the target site. The plurality of fixing elements may be configured to oppose disengagement from the tissue. The fixing elements may each comprise a tine, barb or hook. The fixing elements may be configured to directly couple the occluder member (or a body thereof) to tissue at the target site at the locations of the fixing elements. The plurality of fixing elements may comprise a first set of fixing elements and a second set of fixing elements. The first set of fixing elements may oppose the second set of fixing elements. A tangential component of the fixing direction of the first set of fixing elements may oppose a tangential component of the fixing direction of the second set of fixing elements. The first set of fixing elements may extend in a clockwise direction. The second set of fixing elements may extend in an anticlockwise direction. The second set of fixing elements may be displaced from the first set of fixing elements. The second set of fixing elements may be displaced from the first set of fixing elements in the longitudinal direction.

According to a further aspect of the disclosure there is provided a medical device for treating a target site, the device comprising: a body; and one or more electrodes coupled to the body, wherein the one or more electrodes are electrically insulated from the body, arranged to interface with tissue at the target site and arranged to receive energy to cause the medical device to be bonded to the tissue.

The body may comprise an occluding member. The one or more electrodes may be coupled to the occluding member. The one or more electrodes may be electrically insulated from the occluding member.

One or more electrical leads may be coupled to the one or more electrodes for delivering energy to the one or more electrodes. The one or more electrical leads may each comprise a sacrificial portion. The sacrificial portion may provide a point for detaching the one or more electrical leads from the one or more electrodes.

The one or more electrodes may be arranged to interface with tissue at the target site. The one or more electrodes may be configured to receive energy to cause the one or more electrodes to be heated such that the medical device is bonded to the tissue by the one or more electrodes. The one or more electrodes may each comprise an insulating coating.

The one or more electrodes may each comprise a first side in contact with the body. The one or more electrodes may each comprise a second side configured to interface with the tissue. At least a portion of the second side may be free of insulating material. The body may comprise an occlusive braided fabric. The one or more electrodes may each comprise a wire segment. The wire segment may be wrapped around one or more strands of the braided fabric.

According to a further aspect of the disclosure there is provided a medical device for occluding a left atrial appendage, LAA, the medical device comprising: a proximal occluding member, which may be sized and shaped to cover an ostium of the LAA in an implanted condition of the medical device; a distal member coupled to the proximal occluding member and configured to be positioned within the LAA; and a tether having a first end coupled to the distal member, and a second end opposite the first end, the second end of the tether including a tether anchor configured to pierce tissue forming a distal wall of the LAA.

The tether anchor may include one or more tines or pincers. The tether anchor may include one or more hooks. The tether anchor may include a helical member configured to screw into the distal wall of the LAA. The proximal occluding member may form a disc. In an implanted condition of the medical device the tether may be substantially straight between the first end of the tether and the second end of the tether. In an implanted condition of the medical device, the tether may have a serpentine shape between the first end of the tether and the second end of the tether. The medical device may also comprise a seal member positioned on the tether proximal to the tether anchor, such that in the implanted condition of the medical device, the seal member abuts the distal wall of the LAA.

According to a further aspect of the disclosure there is provided a medical device for treating a target site having an opening, the device comprising a single occlusive member for occluding the opening, wherein the occlusive member is a disc, and wherein the occlusive member is the only portion of the device provided for attaching to the target site. The medical device may be a left atrial appendage occluder and the target site may be an ostium of a left atrial appendage.

According to a further aspect of the disclosure there is provided a medical device for treating a target site having an opening, the device comprising an occlusive member for occluding the opening and an expandable balloon that is releasably coupled to the occlusive member such that causing expansion of the expandable balloon causes the occlusive member to expand from a delivery configuration to a deployed configuration.

The occlusive member may comprise a disc. The medical device may be a left atrial appendage occluder. The target site may be an ostium of a left atrial appendage.

In general, a feature disclosed in relation to one aspect or embodiment may be provided in combination with a feature of a different aspect or embodiment.

### BRIEF DESCRIPTION OF THE DRAWINGS

FIG. 1 illustrates a known medical device used for the occlusion of abnormalities such as a left atrial appendage, LAA.
FIG. 2 illustrates a schematic diagram of a delivery system.
FIG. 3 illustrates a medical device in a deployed configuration occluding a left atrial appendage.
FIG. 4 illustrates a plan view of a medical device for treating a target site.
FIG. 5 illustrates an isometric perspective view of a medical device for treating a target site.
FIG. 6 illustrates a side view of a medical device for treating a target site.
FIG. 7 illustrates another medical device that is generally similar to those described with reference to FIG. 3 to FIG. 5.
FIG. 8A illustrates another medical device that is similar to the medical device described with reference to FIG. 7.
FIG. 8B illustrates another medical device attached to a delivery device comprising a balloon.
FIG. 8C illustrates another medical device comprising a plurality of electrodes attached to a delivery device comprising electrical leads.
FIG. 8D illustrates a delivery device that differs from the arrangement described with reference to FIG. 8B in that the delivery device comprises struts instead of a balloon.
FIG. 9 illustrates a medical device comprising a plurality of electrodes attached to a delivery device comprising electrical leads.
FIG. 10 illustrates a flow diagram of an exemplary method for deploying a medical device at a target site such as an ostium of a LAA.
FIG. 11A illustrates a medical device comprising a lobe in addition to a disc.
FIG. 11B illustrates a medical device comprising a tether in addition to a disc and lobe.
FIG. 12A illustrates a medical device comprising a single disc occluder with electrodes.
FIG. 12B illustrates a medical device comprising a proximal disc and a distal lobe with electrodes.
FIG. 13A illustrates an angled side view of a medical device similar to that described with reference to FIG. 1 and further comprising an electrode. And
FIG. 13B illustrates a plan view of the medical device of FIG. 13A.

### DETAILED DESCRIPTION OF THE DISCLOSURE

The present disclosure relates generally to medical devices that are used in the human body. Specifically, the present disclosure provides a delivery device for delivering, deploying, and installing an occluder at a target site (e.g., a tissue defect, such as a hole through tissue at the target site).

The disclosed embodiments may lead to more consistent and improved patient outcomes. It is contemplated, however, that the described features and methods of the present disclosure as described herein may be incorporated into any number of systems as would be appreciated by one of ordinary skill in the art based on the disclosure herein.

Although the exemplary embodiment of the medical device is described as treating a target site including a hole through the subject tissue, such as an atrial septal defect (ASD), a ventricular septal defect (VSD), a patent foramen ovale (PFO), or a left atrial appendage (LAA), the medical device may be configured to treat any target site that could benefit from occlusion, such as an abnormality, a vessel, an organ, an opening, a chamber, a channel, a hole, a cavity, or the like, located anywhere in the body. Other physiological conditions in the body occur where it is also desirous to occlude a vessel or other passageway to prevent blood flow into or therethrough. These device embodiments may be used anywhere in the vasculature where the anatomical conditions are appropriate for the design.

As used herein, the term "proximal" refers to a part of the medical device or the delivery device that is closest to the operator, and the term "distal" refers to a part of the medical device or the delivery device that is farther from the operator at any given time as the medical device is being delivered through the delivery device. In addition, the terms "deployed," "installed," "secured," and "implanted" may be used interchangeably herein.

The medical device may include one or more layers of occlusive material, wherein each layer may be comprised of any material that is configured to substantially preclude or occlude the flow of blood so as to facilitate thrombosis. As used herein, "substantially preclude or occlude flow" shall mean, functionally, that blood flow may occur for a short time, but that the body's clotting mechanism or protein or other body deposits on the occlusive material results in occlusion or flow stoppage after this initial period.

The present disclosure now will be described more fully hereinafter with reference to the accompanying drawings, in which some, but not all embodiments of the disclosure are shown. Indeed, this disclosure may be embodied in many different forms and should not be construed as limited to the embodiments set forth herein; rather, these embodiments are provided so that this disclosure will satisfy applicable legal requirements. Like numbers refer to like elements throughout.

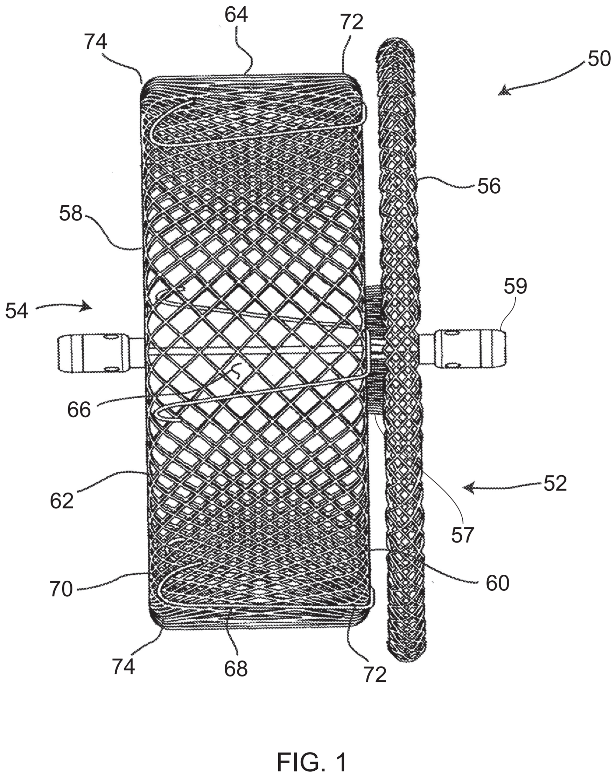

FIG. 1 illustrates a known medical device used for the occlusion of abnormalities such as a left atrial appendage, LAA. The device is generally in the form of a proximal disc 56 coupled to a distal lobe 58 by a connecting portion 57. When deployed at an LAA target site, the proximal disc 56 occludes an ostium of the LAA and the lobe is fixed in place within a cavity of the LAA.

The medical device 50 includes a proximal end 52 and a distal end 54. The proximal disc 56 is provided at proximal end 52 and the distal lobe 58 is provided at the distal end 54. The lobe 58 has a proximal edge 60 (also referred to as a proximal face), a distal edge 62 (also referred to as a distal face), and a middle or central portion 64 that define a cavity 66. As used herein, the term "lobe" may refer to a generally cylindrical shape having a meaningful length (or height) compared to the diameter of one or both faces of the cylinder shape, although it should be understood that the shape need not be perfectly cylindrical. In other words, a disc may not be considered a lobe. The medical device 50 also includes stabilizing wires 68 secured to a radially outer or circumferential surface of middle portion 64. The stabilizing wires 68 terminate in a hook 70 at free ends thereof, and thereby facilitate retention of the medical device 50 at a target site and preventing the medical device 50 from becoming dislodged from the target site after deployment.

In this known medical device 50, proximal edge 60 and distal edge 62 adjoin middle portion 64 at a first relatively blunt or sharp (e.g., non-rounded) transition 72 and a second blunt transition 74, respectively. First blunt transition 72 connects proximal edge 60 to middle portion 64 by an approximately 90 degree angle. Likewise, second blunt transition 74 connects distal edge 62 to middle portion 64 by an approximately 90 degree angle. First blunt transition 72 and second blunt transition 74 partially define a generally rectangular cross section to lobe 58, leading to relatively blunt circumferential edges of the device and relatively high radial force applied to the surrounding tissue.

The medical device 50 further comprises a proximal connecting member 59, or hub, for coupling the medical device 50 to a delivery system during deployment.

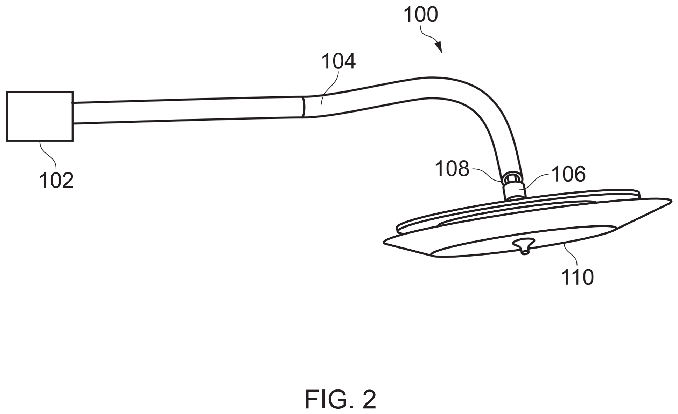

FIG. 2 illustrates a schematic diagram of a delivery system 100. Delivery system 100 includes a delivery device 102 including a catheter 104 and a coupling member 106 configured to couple a distal end of a delivery cable 108 to a medical device 110 (which may be any of the occluders described herein) for facilitating the deployment of medical device 110 at a target site. For example, the coupling member may be configured to engage with the proximal connecting member of a medical device such as that described previously with reference to FIG. 1. The medical device 110 may be deployed to treat the target site, and, in the example embodiment, is an occlusion device ("occluder").

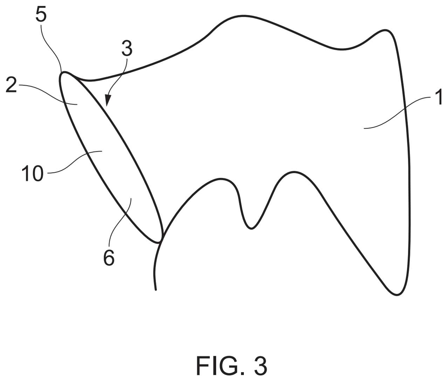

FIG. 3 illustrates a medical device 10 in a deployed configuration occluding a left atrial appendage, LAA, 1. The medical device 10 comprises a single occlusive member 2 sized and arranged to be positioned at an ostium 3 of the LAA 1 in the deployed condition of the medical device. The occlusive member 2 is arranged to at least partially occlude the opening of the ostium and is also the only portion of the medical device 10 that is provided to be affixed to the LAA. The single occlusive member 2 is provided at the ostium and does not substantially extend into the cavity of the LAA 1.

The occlusive member 2 provides a combined occlusive and fixing portion of the medical device 10. It will be appreciated that the medical device 10 may consist of a LAA closure device that has a disc only, rather than a lobe and a disc as described previously reference to the known device of FIG. 1. The omission of a lobe within the LAA cavity enables the medical device 10 to avoid interacting with the tissue of the LAA itself, which is typically thin, irregular, and highly variable in shape. Avoiding this interaction may reduce complications of LAA closure (e.g. pericardial effusions from perforating the thin LAA tissue or residual leaks from irregular tissue/shapes) and/or make the procedure simpler by avoiding the need to consider the shape of the LAA and how the device will sit within the patient's particular anatomy. In this way, the stroke risk in patients with atrial fibrillation may be reduced by the provision of the medical device 10.

The occlusive member 2 is a disc-shaped portion that provides a closed disc, in this example. The disc-shaped portion is substantially planar and may have a substantially circular shape as shown in FIG. 3, or alternatively may be an oval shape. The disc-shaped portion may be provided as a single layer disc as shown in FIG. 3, or alternatively may be a multi-layer disc. The disc-shaped portion may be flat, as shown in FIG. 3, or alternatively have a more substantial thickness in a deployed configuration of the medical device. For example, the disc-shaped portion may be cylindrical without departing from being disc-shaped. The cylindrical disc-shaped portion may have a thickness of less than one of 1mm, 2 mm, 5mm or 10 mm, for example.

The occlusive member 2 may comprise an occlusive braided fabric 6 formed of shape-memory material such as nitinol. In other examples, the occlusive member 2 may comprise other fabric (such as PET) or tissue 6 (such as a pericardial patch). The occlusive braided fabric 6 may be bounded by a frame 5, which may also be formed of shape-memory material. The frame 5 may be provided around a periphery of the occlusive member 2 to bound the occlusive braided fabric 6. If occlusive member 6 comprises an occlusive braided metal fabric 6, the frame 5 may be unnecessary as the occlusive braided metal fabric 6 may have enough structural integrity without needing an additional frame 5. However, if the occlusive member 2 comprises a non-metal fabric or tissue 6, a separate frame 5 may be particularly useful in helping the non-metal fabric or tissue 6 in maintaining a desired shape - however even if the occlusive member 2 comprises a non-metal fabric or tissue 6, the frame 5 may not be needed. For example, in some examples described below, the delivery system includes supports that splay outwardly. That is one example in which the frame 5 may not be needed for embodiments with non-metal fabric or tissue 6. In some examples, instead of (or in addition to) the frame 5 providing structural support for the fabric (metal or non-metal) and/or tissue 6, the frame 5 may serve as an attachment point for one or more anchors, including any of the anchors (or fixing elements, barbs, hooks, *etc.*) described below. It should be understood that these other alternative material options and frame options apply to occluder members described herein that are configured to cover an ostium of the LAA.

The medical device 10 is intended for long term use, as opposed to temporary use during a clinical procedure. As such, the occlusive member 2 comprises or consists of a braided fabric shape formed of a shape memory alloy, such as nitinol, so that the occlusive member 2 may self-expand to cover the ostium 3 of the LAA 1. In this sense, long term use may be considered to be a period of greater than one or three days, for example, but in practice a device may remain in a patient for many years. The material from which the occluder is formed or coated may be selected to promote long term use by, for example promoting endothelial growth over the device after implantation at the target site. In such examples, the material may be selected to be non-thrombogenic, flexible and durable to withstand long-term motion and fatigue, possibly over decades of use. For example, the material may comprise a fluoropolymer such as poly(vinylidene fluoride-co-hexafluoropropylene) (PVDF-HFP). As noted above, the occlusive member 2 may instead consist of a non-metal fabric or tissue, with or without an additional frame 5 (which may be a self-expanding frame 5).

The medical device 10 may be attached to the ostium using fixing elements (not shown) of the medical device 10, or alternatively by applying an external fixing means for attaching the medical device in situ, such as an adhesive or using sutures.

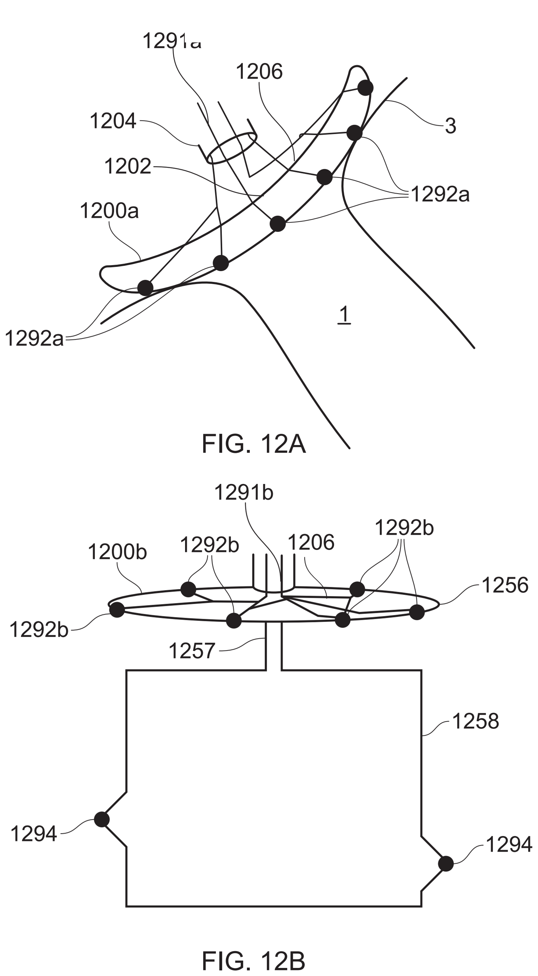

The medical device 10 could be anchored to tissue at the ostium 3 in a number of ways. For example, the medical device 10 may be provided with stabilizing wires as described previously with reference to FIG. 1, or other hooks. The stabilizing wires or hooks may be attached to the occlusive braided fabric 6 or frame 5 of the occlusive member 2. Alternatively, the medical device 10 may be anchored at the edge of the occlusive member 2 using a tissue adhesive, sutures, or heated electrodes as described further below with reference to FIG. 12A.

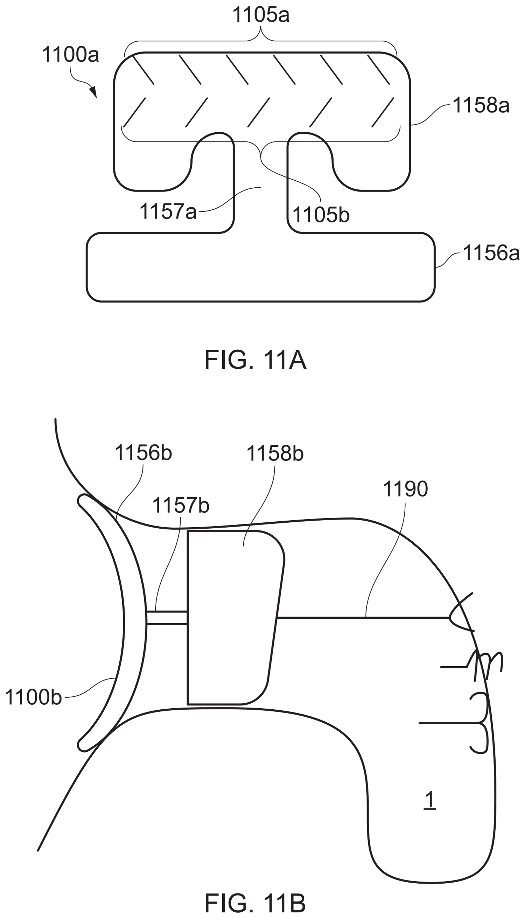

According to a further approach, a tether (not shown) may be attached to the occlusive member 2 (*e.g.* a distal or proximal surface thereof) to anchor the medical device 10 to a distal wall of the LAA. Such a device, further including an intervening lobe, is described further below with reference to FIG. 11B.

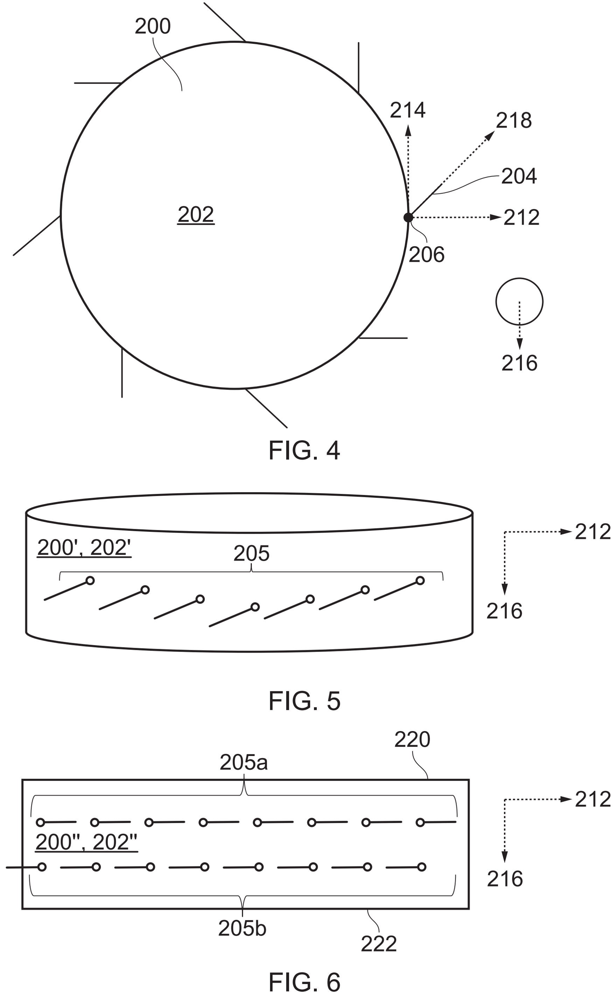

FIG. 4 illustrates a plan view of a medical device 200 for treating a target site (not shown). The medical device 200 comprises a body 202 and a plurality of fixing elements 204, which may also be referred to as anchors, for coupling the body 202 to the target site. The body 202 may provide an occlusive member, such as a single disc, as described previously with reference to FIG. 3.

In this example, the plurality of fixing elements 204 is arranged around a circumference or periphery of the body 202 of the medical device 200. Preferably, the fixing elements 204 have means to resist disengagement from the target site. The fixing elements 204 may each comprise a tine, barb, hook or other element for piercing (or otherwise frictionally engaging) the tissue at the target site to affix the medical device 200 to the target site. In this way, a permanent or long-term bond between the medical device and the target site may be achieved.

The geometry of the body 202 may be described with reference to a cylindrical polar coordinate system having a radial direction, a circumferential direction and a longitudinal direction. More generally, to avoid any implication that the geometry of the device is circular or oval-shaped, for example, the longitudinal direction may be referred to as an axial direction 216, the radial direction may be referred to as an outward direction 212 and a direction taken at a tangent at a point on the perimeter of the body 202 may be referred to as a tangential direction 214.

Each fixing element 204 extends from the body 202 of the medical device 200 at a respective fixing location 206 at an edge of the body 202 of the medical device 200. The fixing elements 204 are configured to directly couple the body 202 to the target site at the fixing locations 206 of the respective fixing elements 204. Each fixing element 204 also extends in a fixing direction 218 with respect to the location 206. The fixing direction 218 has an outward component, extending in an outward direction 212 from the body 202 at the fixing location 206, and a tangential component, extending in a tangential direction 214 to the body 202 at the fixing location 206.

FIG. 5 illustrates an isometric perspective view of a medical device 200' for treating a target site (not shown). The medical device 200' is generally similar to that described previously with reference to FIG. 4. In this example, a plurality of fixing elements 205 are aligned in the axial direction 216 as a single row along a body 202' of the medical device 200'. The fixing elements 205 are spaced apart from one another around a perimeter of the body 202'. Although the fixing elements 205 shown in Fig. 5 may appear to be at different heights, because the fixing elements 205 are positioned on a rounded surface, each fixing element 205 is actually positioned at substantially the same height in the axial direction 216.

FIG. 6 illustrates an side view of a medical device 200" for treating a target site (not shown). The body 202" of the medical device 200" extends along the axial direction 216 between a proximal face 220 and a distal face 222.

The medical device 200" is generally similar to that described previously with reference to FIG. 4. However, in the example in FIG. 6, a first set of fixing elements 205a is aligned in the axial direction 216 as a first row, and a second set of fixing elements 205b is aligned in the axial direction 216 as a second row. The first set of fixing elements 205a is a different distance from a proximal face 220 of the body 202" than is the second set of fixing elements 205b. The first set of fixing elements 205a opposes the second set of fixing elements 205b in that a tangential component of the fixing direction of a fixing element of the first set of fixing elements 205a opposes a tangential component of the fixing direction of a fixing element of the second set of fixing elements 205b that is at a corresponding position along the perimeter of the medical device 200". In this example, whereas the first set of fixing elements 205a extends around the perimeter of the body 202"' of the device with engagement ends of the fixing elements 205a facing in an anti-clockwise direction, the second set of fixing elements 205b extends around the perimeter of the body 202"' of the device with engagement ends of the fixing elements 205b facing in a clockwise direction. Although the fixing elements 205 of Fig. 5 and the fixing elements 205a, 205b of Fig. 6 are both shown in a generally straight line, it should be understood that the fixing elements may be provided in other configurations, including staggered and other configurations.

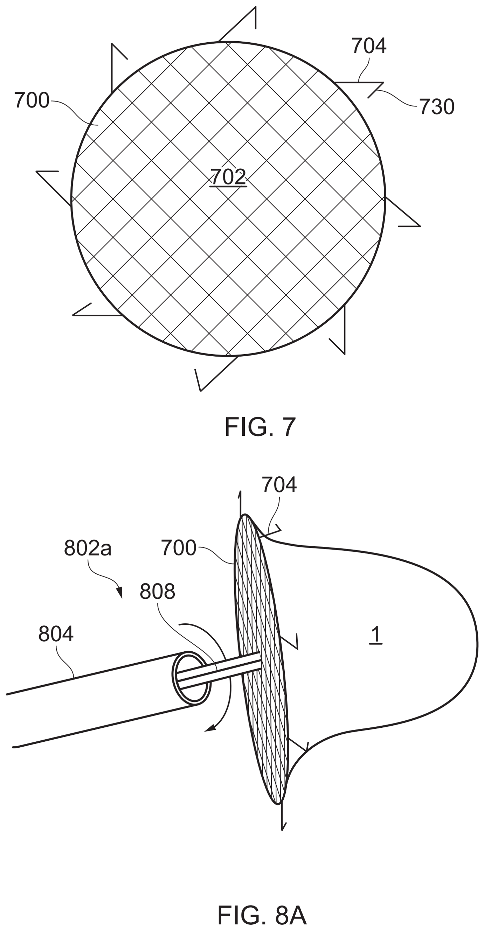

FIG. 7 illustrates a medical device 700 that is generally similar to those described previously with reference to FIG. 3 to FIG. 5. In this example, the medical device 700 comprises a plurality of fixing elements 704. Each fixing element 704 comprises a hooked engagement end 730 for piercing tissue at the target site and resisting disengagement from the target site. Engagement ends of the hooks face in a clockwise direction in this example. As such, a fixing direction of the fixing elements 704 is angled with respect to the single occlusive member 702 such that the fixing elements 704 are arranged to dig into tissue at the target site when the medical device 700 is rotated clockwise is adjacent to or pressed against tissue at the target site. In this way, the hooked ends resist disengagement from the target site due to rotation in the anticlockwise direction. In some examples, the fixing elements 704 may be or include barbs or barb-like features such that, after the fixing elements 704 are driven into engagement with tissue, they are unlikely or unable to be easily disengaged with the tissue.

In this example, the single occlusive member is provided as a disc formed of a braid of wires, which may be provided by a shape memory alloy.

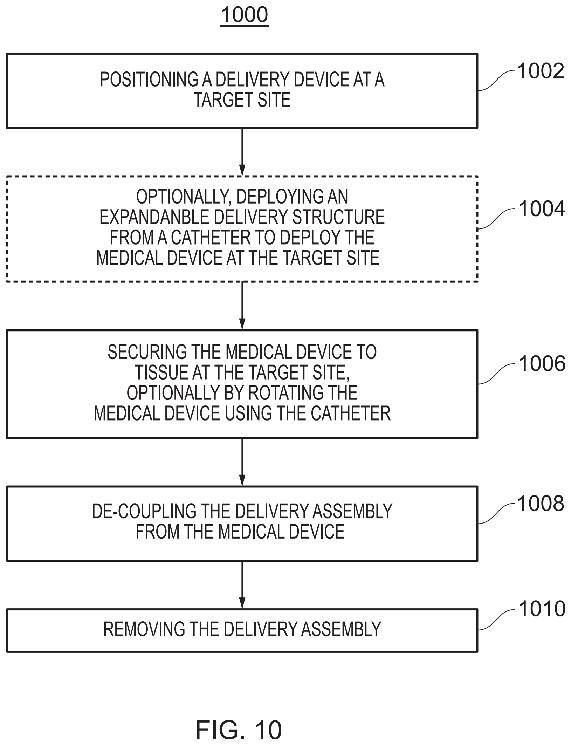

Figures 8A to 8D each illustrate a medical device positioned at a target site and coupled to a delivery mechanism. In these examples, the target site is an ostium of a left atrial appendage. A method for providing a medical device at a target site is described further below with reference to FIG. 10.

In FIG. 8A, the medical device 700 is similar to the medical device described previously with reference to FIG. 7. The medical device 700 is coupled to a delivery device 802a. The delivery device is similar to that described previously with reference to FIG. 2 and includes a catheter 804 and a delivery cable 808. The delivery cable 808 is configured to be coupled to the medical device 700 using, for example, a coupling member (not shown). In this way, the delivery cable 808 may be rotated in order to cause a corresponding rotation of the medical device 700. The rotation of the medical device 700 at the target site causes the fixing elements 704 of the medical device 700 to pierce the tissue at the target site and cause the medical device 700 to be fixed in place at the target site. In some examples, if delivery cable 808 is threadedly coupled to the medical device 700, clockwise rotation may tend to thread the delivery cable 808 onto the medical device 700 for coupling, and anti-clockwise rotation may tend to unthread the delivery cable 808 from the medical device 700 for decoupling. In such examples, it may be preferable for the fixing elements 704 to have terminal ends that point in the clockwise direction, such that clockwise rotation of the medical device 700 tends to engage the fixing elements 704 with tissue. This may be preferable to the alternative in which the terminal ends of the fixing elements 704 point in the anti-clockwise direction, as anti-clockwise rotation of the delivery cable 808 may tend to disconnect the delivery cable 808 from the medical device 700 when trying to engage the fixing elements 704 with the tissue. In other words, if a threaded delivery cable 808 is included, it may be preferable for the terminal ends of the fixing elements 704 to point in the same rotational direction as the delivery cable 808 rotates to connect to the medical device 700. However, this is merely one option, and in other examples, the terminal ends of the fixing elements 704 may point in the same rotational direction as the delivery cable 808 rotates to disconnect from the medical device 700.

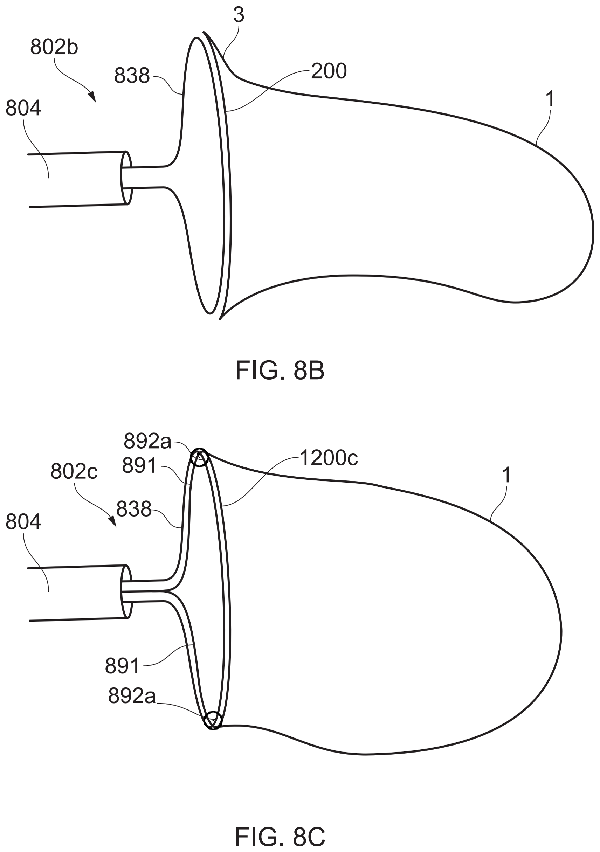

FIG. 8B illustrates medical device 200 attached to a delivery device 802b. In general, the medical device 200 may be similar to or the same as that shown and described in connection with FIG. 4, or may take the form of any medical device described herein, and the medical device 200 may be removably attached to the delivery device 802b, for example by sutures or adhesive. The delivery device 802b includes an inflatable balloon 838. The inflatable balloon 838 may be attached to a distal portion of a delivery cable (not shown). For example, the balloon may be formed on the delivery cable with the delivery cable extending through the balloon and beyond the distal end of the balloon. In such example, the delivery cable may have a threaded distal end that reversibly couples to the medical device 200. The inflatable balloon 838 may be a mushroom-shaped or cylinder-shaped balloon. Prior to delivery, the medical device 200 may be mounted on the distal end of the balloon 838 (and/or to a distal end of the delivery cable) and then collapsed with the balloon onto the delivery catheter 802b. In some examples, the medical device 200 may be removably coupled to the balloon 838, for example via sutures, adhesives (including weak adhesives), or by reversible mechanical couplers, such as clamps (not shown), attached to the balloon 838 can close and open to grip or release the medical device 200. If the medical device 200 is coupled to the balloon 838 via sutures, the balloon 838 may include lumens to receive the sutures therethrough without piercing the balloon 838, or the balloon 838 may include a covering (e.g. a fabric covering) which may receive the sutures. When in position at the LAA 1, the balloon 838 can be inflated, oriented properly and then advanced so that the balloon 838 pushes the device up against the LAA ostium 3. The expansion of the balloon 838 causes the medical device 200 to expand from a delivery configuration to a deployed configuration. The pressure of the medical device 200 abutting the ostium may cause anchors (not shown) of the medical device 200 to engage the tissue at the target site. Alternatively or additionally, the medical device 200 may be rotated to engage the anchors of the medical device 200 as described previously. The exterior of the balloon may incorporate tubes for the injection of a tissue adhesive at the edge of the medical device 200. The tubes extend into the delivery catheter to allow adhesive to be delivered. In this way, the balloon may be used to affix a medical device 200 to tissue at the target site without integral anchors. Once the medical device 200 is anchored, the balloon may be deflated, detached from the medical device 200 (*e.g.* be using a suture cutter, overcoming force of a weak adhesive, opening a clamp attached to the balloon, *etc.*) and removed.

FIG. 8C is discussed further below in relation to FIG. 12A.

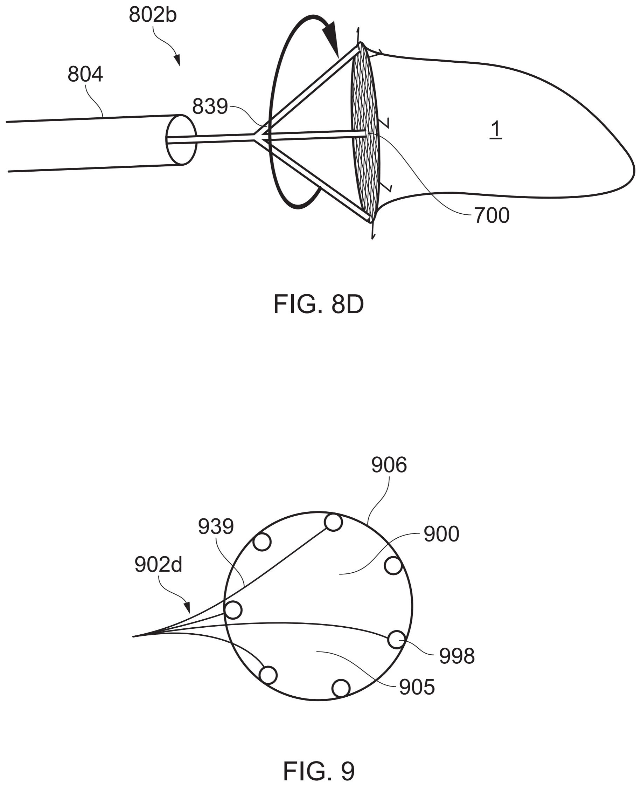

Turning to FIG. 8D, which illustrates a delivery device 802b that differs from the arrangement described with reference to FIG. 8B in that the delivery device 802b comprises struts 839 instead of a balloon. For example, struts comprising a shape memory alloy such as nitinol may be used to form a funnel shape during device deployment. The struts may be formed of tubes or wires that are thicker than the wires of the braided fabric.

Prior to delivery, a proximal end of the medical device 700 is attached to distal ends of the struts 839 and then collapsed in a delivery sheath of the catheter 804. The attachment between the struts 839 and the medical device 700 may be achieved by sutures or adhesive, for example. When in position at the target site, the sheath of the catheter 804 may be retracted to expand the struts into the funnel shape, also expanding the medical device 700. Then, the medical device 700 may be pressed against the LAA ostium and anchored in a similar manner as described previously with reference to FIG. 8B, for example by rotating the delivery cable to rotate the struts 839 in unison, thereby rotating the medical device 700. In some example, instead of (or in addition to) anchoring the medical device 700 with hooks or similar fixing elements, radiofrequency ("RF") energy may be applied to fix the medical device 700 to the tissue in contact with the medical device. The struts 839 may then be detached from the medical device 700, retracted into the sheath of the catheter 804, and removed from the patient. In some examples, the struts 839 may be individually rotatable with each strut 839 screwing into corresponding threaded element of the medical device 700, such that the struts 839 may be individually rotated to connect and disconnect each strut 839 to the medical device 700.

FIG. 9 illustrates a medical device 900 attached to a portion of a delivery device 902d. The medical device 900 may be of the type described previously with reference to FIG. 3 and may comprise a frame 906 bounding a fabric of braided metal or non-metal wires 905, or a frame 906 bounding tissue 905, although in some examples the frame 906 may be omitted similar to as described above in connection with Fig. 3. The delivery device 902d comprises struts 939, or wires, that are detachably coupled to the medical device 900 at a plurality of contact points 998. The coupling between the struts 939 and the medical device 900 may be achieved similar as described in connection with Fig. 8D, including by sutures or adhesive, for example.

The struts 939, or wires, form a frame of a funnel or cone shape during device deployment. In some examples, material may be provided between the struts 939 so that the delivery device 902d provides an enclosed volume defined by the struts and the medical device 900.

FIG. 10 illustrates a flow diagram of an exemplary method 1000 for deploying a medical device, such as a medical device described herein comprising an occluding disc, at a target site such as an ostium of an LAA. The method 1000 includes positioning 1002 a delivery device at the target site. The delivery device includes an expandable installation structure, such as a balloon or self-expanding installation frame, extending between a proximal end and a distal end. The delivery device further includes a delivery cable having a distal end coupled to the proximal end of the installation structure. In a deployment configuration, the disc of the medical device is releasably coupled to the distal end of the installation structure.

Method 1000 may also include deploying 1004 the installation structure from a delivery catheter. Deploying 1004 the installation structure includes expanding the installation structure from a constricted delivery configuration to an expanded deployment configuration and substantially simultaneously expanding the disc coupled to the installation structure. In some instances, deploying 1004 includes proximally retracting the delivery catheter, and/or distally advancing the delivery cable through the delivery catheter.

Method 1000 further includes securing 1006 the medical device to tissue at the target site. Securing 1008 may include affixing, suturing, adhering, bonding or stapling the disc to the tissue. In some embodiments, securing 1008 includes rotating the medical device by rotating the delivery cable and installation structure to cause fixing elements of the medical device to engage with tissue at the target site.

The disc is decoupled 1008 from the delivery device and the delivery device can be removed 1010, leaving the medical device in situ. In some embodiments, de-coupling 1010 may include trimming one or more threads of suture material coupling the disc to the distal end of the disc installation frame, for example using a separate cutting tool to cut the threads or energy application tool (e.g. cautery tool) to burn through the threads.

Method 1000 may include additional, alternative, and/or fewer steps, including those described herein.

In some embodiments, method 1000 may include coupling the disc to the installation structure prior to positioning 1002, which may include threading one or more threads of suture material about a circumference of the distal end of the installation structure and through the disc.

Moreover, in some embodiments, method 1000 may include verifying a position of the disc delivery device prior to securing 1008 the disc. This verifying may include, for example, performing contrast injection(s) under fluoroscopy, to verify the device is in the proper location and the target site (and/or the ostium thereof) is covered prior to securing the disc. Additionally or alternatively, verifying may include detecting any radiopaque material coupled to and/or integrated into the disc. In some embodiments, method 1000 may further include withdrawing the disc delivery device from the target site while leaving the disc secured to the tissue at the target site.

FIG. 11A illustrates a medical device 1100a comprising a lobe 1158a in addition to a disc 1156a. The disc 1156a is coupled to the lobe 1158a by a connecting portion 1157a and the device may be implemented, for example, as an occluder for a left atrial appendage similar to that described with reference to FIG. 1. The medical device 1100a differs from the device described previously with reference to FIG. 1 in that, for example, the stabilising wires of the device of FIG. 1 are replaced with fixing elements 1105a, 1105b, or anchors, as described previously with reference to FIG. 6. Alternatively, another of the previously described arrangements of fixing elements may be provided on the distal lobe 1158a. For example, the distal lobe 1158a of the device 1100a may provide the body of the medical device described previously with reference to figures 4 to 6, for example.

In this example, the plurality of fixing elements 1105a, 1105b may each comprise a hooked end for piercing (or otherwise frictionally engaging) tissue at the target site and resisting disengagement from the target site. A fixing direction of the fixing elements 1105a, 1105b is angled with respect to the distal lobe 1158a such that the fixing elements are arranged to dig into tissue at the target site when the medical device 1100a is rotated. For example, in use, rotating the medical device 1100a (and particularly distal lobe 1158a) in a first rotational direction may allow for fixing elements 1105a to pierce (or otherwise frictionally engage) tissue, while rotating the medical device 1100a (and particularly distal lobe 1158a) in a second rotational direction opposite the first rotational direction may allow for fixing elements 1105b to pierce (or otherwise frictionally engage) tissue. This may be achieved by having the hooks or other piercing or engaging ends of the fixing elements 1105a oriented substantially in the opposite direction as fixing elements 1105b. In some examples, during use, the medical device 1100a (and particularly distal lobe 1158a) may be rotated in the first rotational direction a first amount to engage fixing elements 1105a with tissue, and thereafter the medical device 1100a (and particularly distal lobe 1158a) may be rotated in the second rotational direction a second amount less than the first amount to engage fixing elements 1105b with tissue without disengaging the fixing elements 1105a. For example, the first set of fixing elements can be engaged with tissue by twisting or rotating the medical device 1100a about 3-4 mm (so that the fixing elements travel a distance of about 3-4 mm), and the other set of fixing elements may be subsequently engaged with tissue by twisting or rotating the medical device about 1-2 mm (so that the fixing elements travel a distance of about 1-2 mm) in the other direction. In this way, the two sets of hooked ends resist disengagement from the target site since each set of hooks resists disengagement in different ones of the two rotational directions. Other means for fixing the distal lobe 1158a in place at the target site may be used in addition to the fixing elements 1105a, 1105b.

By providing the fixing elements on the distal lobe 1158a, the medical device 1100a may be fixedly attached to tissue within a cavity of an LAA. For example, the lobe could be rotated (e.g. in opposite directions sequentially) during or after deployment of the medical device to engage anchors, as described above.

In addition, or alternatively, fixing elements such as those described previously with reference to figures 4 to 6 may be provided on the proximal disc 1156a of the medical device 1100a.

FIG. 11B illustrates a medical device 1100b for treating an LAA. The medical device 1100b comprises a proximal disc 1156b, a lobe 1158b and a distal tether 1190. A distal end of the proximal disc 1156b is coupled to a proximal end of the lobe 1158b by a connecting portion 1157b. The distal tether 1190 extends from a distal end of the lobe 1158b. The distal tether 1190 has a distal end configured to engage tissue in a backwall of the LAA 1. The distal tether 1190 end comprises an engagement means for engaging with and being retained by the tissue of the LAA 1. The engagement means may be provided by a helix, grasper, pincer or hooks as shown in the alternatives in FIG. 11B (which alternatives are shown disconnected from a main tether body and the medical device for purposes of clarity).

The medical device 1100b may be loaded into a catheter so that the catheter is brought into contact with the backwall of the LAA 1 to engage the distal end of the distal tether 1190 into the backwall of the LAA 1. Subsequently, a sleeve of the catheter is retracted so that the lobe 1158b is deployed within the cavity of the LAA 1 and, subsequently, the proximal disc 1156b of the medical device 1100b is deployed at the ostium of the LAA to occlude the ostium. The medical device 1100b may then be detached from the delivery system, for example by providing a detachable coupling between the delivery system and the proximal surface of the proximal disc 1156b.

FIGs 12A and 12B illustrate medical devices 1200a, 1200b provided with electrodes 1292a, 1292b, 1294 for sealing the respective medical device 1200a, 1200b to tissue at a target site 3.

FIG. 12A illustrates a medical device 1200a comprising a single disc occluder of the type described previously with reference to FIG. 3. In the illustrated example, the medical device 1200a is situated at an ostium 3 of an LAA 1.

The occlusive member 1202 is formed of a fabric of braided wires 1206 formed from a shape memory alloy material. A plurality of electrodes 1292a are arranged around a periphery of the occlusive member 1202 such that the electrodes 1292a abut tissue at the target site when in use.

The electrodes 1292a provide contact points for welding the medical device 1200a to the surrounding tissue. For example, radio frequency energy may be provided to the plurality of electrodes 1292a in order to cause the electrodes to heat up such that the electrodes are bonded to the surrounding tissue by causing localised damage to the surrounding tissue. Alternatively, a direct current could be applied to the electrodes in order to cause heating. Preferably, energy is provided to the electrodes via one or more electrical leads that are coupled to the respective electrodes. In general, the process of application of the heat could vary from a direct bonding application, such as in a heat press, or the heating may provide a triggered healing response that forms more an internal scab, for example, to hold the medical device 1200a to the surrounding tissue.

The electrodes 1292a are not in electrical contact with each other. That is, there is not a galvanic conduction path through the fabric of braided wires 1206 between the electrodes. In some examples, the electrodes may be insulated from each other by providing the fabric of braided wires 1206 from an insulating material. In other examples, the fabric of braided wires 1206 may be made from an electrically conductive material and coated in an insulating layer, such as an enamel layer. In yet a further example, the electrodes 1292a may be partially coated in an insulating material in order to insulate the electrodes 1292a from the fabric of braided wires 1206.

The plurality of electrodes may be provided by segments of wire. For example, the segments of wire forming the electrodes 1292a may be thicker wire than the wire that forms the braided fabric of the occlusive member 1202.

Each electrode 1292a is attached to an electrical lead 1291a that extends into the catheter 1204 for delivering power to the electrodes 1292a. Multiple electrodes 1292a may be connected to the same lead 1291a. In this example, a plurality of electrical leads 1291a is provided. Alternatively, all the electrodes could be connected to a single lead.

Turning to FIG. 8C, a medical device 1200c is illustrated attached to a delivery device 802c. The delivery device is generally as described previously with reference to FIG. 8B. The medical device 1200c is generally as described previously with reference to FIG. 12A and comprises a plurality of electrodes 892a, and each electrode is attached to a corresponding electrical lead 891 that extends into the catheter 804.

The electrical leads 891 may be detachably coupled to the respective electrodes 892a so that once energy has been applied to the electrodes 892a to cause the medical device 1200c to be fixed in place at the target site 3, the electrical leads can be detached from the electrodes 892a and removed from the medical device 1200c. For example, a lower resistance, or weakened, sacrificial portion of the leads may be provided adjacent to the connection between the leads and the plurality of electrodes so that once the electrodes have heated up sufficiently, the leads are burnt through. This may be achieved by applying a pulse of energy that is sufficient to burn through the sacrificial portion of the electrical leads. This pulse of energy may be of a higher intensity, or at a different frequency than a proceeding level of applied energy that causes the plurality of electrodes 892a to heat up. The detached electrical leads may be retracted with a catheter 804 following deployment of the medical device 1200c.

FIG. 12B illustrates a medical device comprising a proximal disc 1256 and a distal lobe 1258. The proximal disc 1256 is coupled to a distal lobe 1258 by a connecting portion 1257. The medical device 1200b may generally relate to the medical device described previously with reference to FIG. 1. However, the medical device comprises a plurality of electrodes 1292b, 1294 to fix the medical device 1200b in situ at a target site instead of stabilising wires.

In the illustrated example, a plurality of electrodes 1292b are provided on the proximal disc, which generally relates to the occlusive member 1202 described previously with reference to FIG. 12A. In this example, a second plurality of electrodes 1294 is provided around a periphery of the distal lobe 1258. The second plurality of electrodes 1294 is arranged to abut tissue within a cavity of a LAA when the medical device 1200b is deployed so that heating the second plurality of electrodes 1294 causes the distal lobe 1258 to be fused with or bonded to the tissue within the cavity and so anchor the medical device 1200b in situ at the target site.

It will be appreciated that other types of anchors or fixing elements may be provided in addition to the electrodes on either the proximal disc 1256 or the distal lobe 1258. As a further alternative, electrodes 1294 may be provided only on the distal lobe 1258 and not the proximal disc 1256. It would be further appreciated that electrodes for welding to adjacent tissue at a target site may be used on medical devices of other geometries.

Returning to the medical device of FIG. 11B, the engagement means may comprise one or more electrodes (not shown), such as the electrodes discussed with reference to FIGs 12A and 12B, for sealing the medical device to tissue. In such examples, the tether may comprise one or more electrical leads coupled to the one or more electrodes. The one or more one or more electrical leads of the tether may be detachably coupled to one or more corresponding electrical leads of a deployment device.

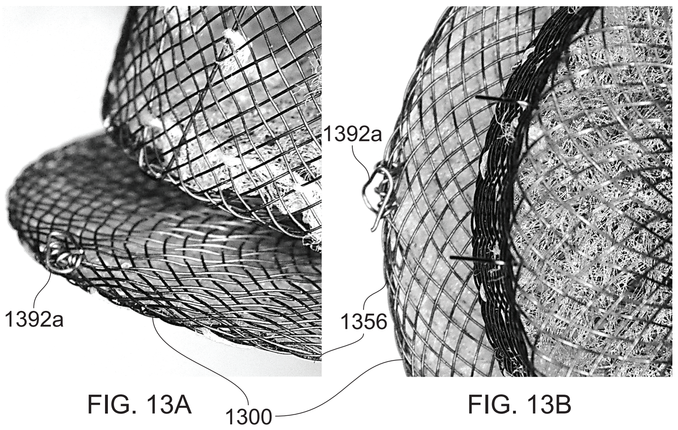

Figures 13A and 13B illustrate various electrodes applied to a medical device 1300. The medical device 1300 may be provided by a device of the type described previously with reference to FIG. 1.

FIGs 13A and 13B illustrate views of an electrode 1392a provided at a periphery of a proximal disc 1356 of the medical device 1300. FIG. 13A illustrates an angled side view of the medical device 1300 and FIG. 13B illustrates a plan view of the medical device 1300.

The proximal disc 1356 is formed of a fabric of braided wires. The electrode 1392a is formed from a segment of electrically conductive wire that is intertwined with the fabric of braided wires of the proximal disc 1356 at an edge of the proximal disc 1356 such that the electrode 1392a contacts tissue adjacent to the proximal disc 1356 when the proximal disc 1356 is in situ at a target site. The electrode 1392a may be operated as described previously with reference to FIGs 12A and 12B. In some examples, a lead wire may be coupled to the electrode and fed through the medical device 1300 so that energy can be applied to the electrode 1392a during deployment of the medical device 1300. The fabric of braided wires may be coated in an enamel to avoid a larger body of the medical device from becoming electrically live when energy is applied to the electrode 1392a. Alternatively, the electrode may be formed from a wire that is coated in an insulating layer, such as an enamel, in order to electrically insulate the electrode 1392a from the fabric of braided wires of the proximal disc 1356. In such examples, the insulating coating of the electrode 1392a may be removed from portions of the electrode 1392a that are arranged to contact the surrounding tissue, or which are not in contact with the fabric of braided wires of the proximal disc 1356.

While embodiments of the present disclosure have been described, it should be understood that various changes, adaptations and modifications may be made therein without departing from the spirit of the disclosure and the scope of the appended claims. Further, all directional references (e.g., upper, lower, upward, downward, left, right, leftward, rightward, top, bottom, above, below, vertical, horizontal, clockwise, and counterclockwise) are only used for identification purposes to aid the reader's understanding of the present disclosure, and do not create limitations, particularly as to the position, orientation, or use of the disclosure. It is intended that all matter contained in the above description or shown in the accompanying drawings shall be interpreted as illustrative only and not limiting. Changes in detail or structure may be made without departing from the spirit of the disclosure as defined in the appended claims.

Many modifications and other embodiments of the disclosure set forth herein will come to mind to one skilled in the art to which this disclosure pertains having the benefit of the teachings presented in the foregoing descriptions and the associated drawings. Therefore, it is to be understood that the disclosure is not to be limited to the specific embodiments described and that modifications and other embodiments are intended to be included within the scope of the appended claims. Although specific terms are employed herein, they are used in a generic and descriptive sense only and not for purposes of limitation.

## Claims

1. A medical device (10, 200, 700) for occluding a left atrial appendage, LAA (1), the medical device comprising a single occlusive member (2, 202, 702) configured to occlude an ostium (3) of the LAA in a deployed condition of the medical device, wherein the occlusive member is a disc-shaped portion, and wherein the medical device is configured to be attached only to the ostium of the LAA.

2. The medical device (10, 200, 700) of claim 1, wherein the occlusive member (2, 202, 702) is a combined occlusive and fixing portion.

3. The medical device (10, 200, 700) of claim 1 or claim 2, wherein the occlusive member (2, 202, 702) comprises a frame (5) and an occlusive braided fabric.

4. The medical device (10, 200, 700) of any preceding claim, wherein the occlusive braided fabric consists of only the single disc-shaped portion.

5. The medical device (10, 200, 700) of any preceding claim, further comprising a plurality of fixing elements (204, 704) for attaching the occlusive member (2, 202, 702) to the ostium (3), wherein the plurality of fixing elements is provided at a periphery of the occlusive member.

6. The medical device (10, 200, 700) of claim 5, wherein each fixing element (204, 704) extends from the occlusive member (2, 202, 702) in a fixing direction (218) and at a respective fixing location (206) on the occlusive member, wherein the fixing direction has an outward component, extending in an outward direction (212) from the occlusive member at the location, and a tangential component, extending in a tangential direction (214) to the occlusive member at the location.

7. The medical device (10, 200, 700) of claim 5 or claim 6, wherein each fixing element (204, 704) is arranged such that rotation of the medical device in a first direction causes the plurality of fixing elements to engage with tissue at the ostium (3).

8. The medical device (10, 200, 700) of any of claims 5 to 7, wherein the plurality of fixing elements (204, 704) is configured to oppose disengagement from tissue at the ostium.

9. The medical device (10, 200, 700) of any of claims 5 to 8, wherein the fixing elements (204, 704) each comprise a tine, barb or hook (730).

10. The medical device (10, 200, 700) of any of claims 5 to 9, wherein the fixing elements (204, 704) are configured to directly couple the occlusive member (2, 202, 702) to tissue at the ostium (3) at the locations of the fixing elements.

11. The medical device (10, 200, 700) of any of claims 5 to 10, wherein the plurality of fixing elements (204, 704) comprises a first set of fixing elements (205a) and a second set of fixing elements (205b), wherein the first set of fixing elements oppose the second set of fixing elements.

12. The medical device (10, 200, 700) of claim 11, wherein a tangential component (214) of the fixing direction (218) of the first set of fixing elements (205a) opposes a tangential component (214) of the fixing direction (218) of the second set of fixing elements (206b).

13. The medical device (10, 200, 700) of claim 12, wherein the first set of fixing elements (205a) extend in a clockwise direction and the second set of fixing elements (205b) extend in an anticlockwise direction.

14. The medical device (10, 200, 700) of claim 13, wherein the second set of fixing elements (205b) is displaced from the first set of fixing elements (205a) in the longitudinal direction (216).

15. The medical device (10, 200, 700) of any preceding claim, further comprising:
an installation structure comprising a self-expanding body (839) extending between a proximal end and a distal end,
wherein, in a deployment configuration of the medical device, the occlusive member is releasably coupled to the distal end of the installation structure.
